# EUROPÄISCHE PATENTANMELDUNG

(11) **EP 0 610 926 A1**
(43) Veröffentlichungstag der Anmeldung: **17.08.1994**
(21) Anmeldenummer: 94102046.3
(22) Anmeldetag: 10.02.1994
(51) Int. Cl.: A61K 7/42, A61K 33/30, A61K 33/24

(54) **Topische Zubereitungen mit einer Schutzwirkung gegenüber Nesseltieren**

(30) Priorität: 11.02.1993 DE 4303983
(71) Anmelder: Beiersdorf Aktiengesellschaft, D-20245 Hamburg (DE)
(72) Erfinder: Hoppe, Udo, Dr., D-22397 Hamburg (DE); Degwert, Joachim, Dr., D-21255 Tostedt (DE); Gers-Barlag, Heiner, Dr., D-25495 Kummerfeld (DE)

(57) **Zusammenfassung**

Topische Formulierungen mit einem Gehalt an
a) anorganischen Mikropigmenten sowie
b) gegebenenfalls zusätzlichen organischen UV-Filtersubstanzen,
c) wobei die Formulierungen Hydrodispersionen darstellen,
d) welche aus einer inneren Lipidphase und einer äußeren wäßrigen Phase bestehen, und
e) welche im wesentlichen frei von Emulgatoren sind, und
f) wobei die anorganischen Mikropigmente bevorzugt in die Lipidphase der Hydrodispersionen eingearbeitet sind,
zum Schutz gegen Nesseltiere.

## Beschreibung

Die vorliegende Erfindung betrifft topische Zubereitungen, insbesondere kosmetische Zubereitungen.

Nesseltiere wie Quallen, Medusen oder Polypen lähmen ihre Beutetiere durch Abschießen giftgefüllter Nesselkapseln aus Nesselzellen. Der Abschußvorgang wird durch mechanische und chemische Stimulation, aber auch von Menschen beim Baden und Tauchen ausgelöst. Dabei kommt es bei ungeschützter Haut zu Verletzungen, gefährlichen Reaktionen und sogar zu Todesfällen. Das Nesselgift wird in die Haut abgegeben und wirkt hämotoxisch, myotoxisch oder neurotoxisch und führt auch zur Sensibilisierung mit der nachfolgenden Gefahr heftiger allergischer Reaktionen. Zur Behandlung dieser Reaktionen und von Verletzungen sind Medikamente (Antihistaminika) erhältlich.

Als Schutzmaßnahmen sind schon Barrieren an Stränden oder auch eine Schutzkleidung für Badende bekannt. Diese Maßnahmen haben aber Nachteile, da auch sogar abgestorbene Tentakelteile noch intakte Nesselzellen tragen und zu den Badenden gelangen, z.B. auch auf die Haut unter der Schutzkleidung. Eine Schutzkleidung ist außerdem sehr lästig, besonders im Sommer und in den Tropen.

Es wurden auch schon kosmetische W/O-Emulsionen hinsichtlich ihrer Schutzwirkung gegenüber Feuerquallen (Cyaneta capillata) an menschlicher Haut untersucht (Heeger, T.; Möller, H.; Mrowietz, U. 1992: Marine Biology 113, 669-678). Die Emulsionen bewirken zwar eine Verringerung der Anzahl Nesselkapselabschüsse, aber sie bieten in der Praxis keinen immer zufriedenstellenden Schutz gegenüber Nesseltieren.

Aufgabe der Erfindung war es daher, Zubereitungen mit verbesserter Schutzwirkung gegenüber Nesseltieren zur Verfügung zu stellen, die die Nachteile des Standes der Technik nicht mehr aufweisen.

Gegenstand der Erfindung ist die Verwendung von topischen Formulierungen mit einem Gehalt an
a) anorganischen Mikropigmenten sowie
b) gegebenenfalls zusätzlichen organischen UV-Filtersubstanzen,
c) wobei die Formulierungen Hydrodispersionen darstellen,
d) welche aus einer inneren Lipidphase und einer äußeren wäßrigen Phase bestehen, und
e) welche im wesentlichen frei von Emulgatoren sind, und
f) wobei die anorganischen Mikropigmente bevorzugt in die Lipidphase der Hydrodispersionen eingearbeitet sind,
zum Schutz gegen Nesseltiere.

Die topisch anzuwendenden Formulierungen können kosmetische oder dermatologische Formulierungen sein. Der Begriff "dermatologische Formulierungen" schließt auch "dermatologische pharmazeutische Formulierungen" und "dermopharmazeutische Formulierungen" mit ein.

Gegenstand der Erfindung sind auch topische Formulierungen mit einem Gehalt an
a) anorganischen Mikropigmenten sowie
b) gegebenenfalls zusätzlichen organischen UV-Filtersubstanzen,
c) wobei die Formulierungen Hydrodispersionen darstellen,
d) welche aus einer inneren Lipidphase und einer äußeren wäßrigen Phase bestehen, und
e) welche im wesentlichen frei von Emulgatoren sind, und
f) wobei die anorganischen Mikropigmente bevorzugt in die Lipidphase der Hydrodispersionen eingearbeitet sind,
zum Schutz gegen Nesseltiere.

Überraschenderweise schützen die erfindungsgemäßen Formulierungen hervorragend vor Angriffen von Nesseltieren, dem Auslösen der Nesselkapseln und vor dem Eindringen der Nesselfäden oder des Nesselgiftes in die Haut. Sie werden dazu vor dem möglichen Kontakt mit den Tieren auf die Haut aufgebracht und zwar in den für topische Präparate üblichen Mengen.

Besonders bevorzugt werden erfindungsgemäße Formulierungen mit einem Gehalt an anorganischen Mikropigmenten, die als UV-Filtersubstanzen dienen können, und einem zusätzlichen Gehalt an organischen UV-Filtersubstanzen. Diese Formulierungen sind zugleich wertvolle Sonnenschutzmittel.

Die erfindungsgemäßen Zubereitungen oder gemäß der Erfindung zu verwendenden Formulierungen zeichnen sich in vorteilhafter Weise auch durch eine hohe Wasserfestigkeit aus, so daß die Schutzwirkung in Wasser und sogar in Salzwasser lange erhalten bleibt.

Hydrodispersionen stellen Dispersionen einer flüssigen, halbfesten oder festen inneren (diskontinuierlichen) Lipidphase in einer äußeren wäßrigen (kontinuierlichen) Phase dar. Die anorganische Mikropigmente können in die Lipidphase und/oder die wäßrige Phase der Hydrodispersion eingearbeitet sein. Bevorzugt werden Hydrodispersionen, die einen deutlichen oder großen Anteil anorganischer Mikropigmente oder einen überwiegenden Anteil oder praktisch die gesamte Menge dieser Mikropigmente in der Lipidphase enthalten.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen, metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Zwar werden in den Schriften EP-OS 456 458, EP-OS 456 459 und EP-OS 456 460 Lichtschutzformulierungen auf der Basis von TiO₂-Pigmenten beschrieben. Emulgatorfreie Systeme sind aber auf solche Weise nicht realisierbar. Auch sind pigmenthaltige Hydrodispersionen der erfindungsgemäßen Art und ihre Verwendung zum Schutz vor Nesseltieren und deren Giftsekret bisher nicht bekannt gewesen.

Überraschend und nicht vorhersehbar war, daß bei Befolgung der hiermit offenbarten Lehre zum technischen Handeln in jeglicher Hinsicht überaus befriedigende Präparate erhältlich sind. Vielmehr war zu erwarten gewesen, daß sich die Mikropigmentpartikel, mangels eines Emulgators, zu Agglomeraten zusammenballen würden.

Ferner war überraschend, daß die Klebrigkeit der Hydrodispersionen bei Befolgung der hiermit offenbarten Lehre zum technischen Handeln drastisch vermindert werden kann.

Schließlich war überraschend, daß der Einsatz von anorganischen Pigmenten in den erfindungsgemäßen Formulierungen nicht zu starker Hauttrockenheit führt, sondern im Gegenteil ein anhaltendes, äußerst angenehmes Hautgefühl verursacht.

Erfindungsgemäße kosmetische und dermatologische Zubereitungen enthalten bevorzugt anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂.

Voraussetzung für die Verwendbarkeit anorganischer Pigmente für die erfindungsgemäßen Zwecke ist natürlich die kosmetische bzw. dermatologische Unbedenklichkeit der zugrundeliegenden Substanzen.

Im wesentlichen unerheblich für die vorliegende Erfindung ist dabei, in welchen Modifikationen solche Metalloxide vorliegen. TiO₂ beispielsweise kommt in der Natur in drei Hauptmodifikationen (Rutil, Anatas und Brookit) vor, welche grundsätzlich alle gleichermaßen geeignet sind. Ähnliches gilt für die Modifikationen der Eisenoxide usw..

Vorteilhaft ist, den Partikeldurchmesser der verwendeten Pigmente kleiner als 100 nm zu wählen.

Es ist besonders vorteilhaft im Sinne der vorliegenden Erfindung, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

n TiO₂ + m (RO)₃Si-R' -> n TiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen T 805 (Fa. Degussa) oder MT 100 T von der Firma TAYCA erhältlich.

Erfindungsgemäße Zubereitungen enthalten bevorzugt Titandioxid als anorganisches Mikropigment und/oder Polyacrylate (Carbomer) als Verdickungsmittel bzw. Gelbildner oder Filmbildner.

Die erfindungsgemäßen topischen, kosmetischen und/oder dermatologischen Formulierungen und insbesondere Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen topischen, kosmetischen und dermatologischen Zubereitungen in der für Kosmetika üblichen Weise auf die Haut und/oder die Haare in ausreichender Menge aufgebracht, zweckmäßigerweise vor dem Baden oder Tauchen.

Besonders bevorzugt sind solche kosmetischen und dermatologischen Zubereitungen, die in der Form eines Sonnenschutzmittels vorliegen. Vorzugsweise enthalten diese zusätzlich mindestens einen UVA-Filter und/oder mindestens einen (weiteren) UVB-Filter und/oder mindestens ein anorganisches Pigment.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate oder Silikonöle.

Die Lipidphase kann vorteilhaft gewählt werden aus folgender Substanzgruppe:
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Besonders bevorzugt ist, die Komponenten der Lipidphase aus der Gruppe der Silikonöle zu wählen.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder
   -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Cellulosederivate, Hydroxypropylmethylcellulose, besonders vorteilhaft aus der Gruppe der Polyacrylate, bevorzugt ein Polyacrylat aus der Gruppe der sogenannten Carbopole (Carbomer), Fa. Goodrich, beispielsweise Carbopole der Typen 980, 981, 1382, 2984, 5984, jeweils einzeln oder in Kombination.

Verdickungsmittel können vorzugsweise in Mengen von 0,1 bis 2,5 Gew.-%, insbesondere 0,7 bis 1,5 Gew.-%, jeweils bezogen auf die gesamte Formulierung enthalten sein.

Die topischen, kosmetischen oder dermatologischen Zubereitungen enthalten anorganische Pigmente z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen. Besonders bevorzugt wird Titandioxid.

Es ist erfindungsgemäß vorteilhaft, außer den anorganischen Pigmenten öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Vorteilhafte öllösliche UVB-Filtersubstanzen sind z.B.:
- 3-Benzylidencampher-Derivate, vorzugsweise
   3-(4-Methylbenzyliden)campher, 3-Benzylidencampher;
- 4-Aminobenzoesäure-Derivate, vorzugsweise
   4-(Dimethylamino)-benzoesäure(2-ethylhexyl)ester,
   4-(Dimethylamino)benzoesäureamylester;
- Ester der Zimtsäure, vorzugsweise
   4-Methoxyzimtsäure(2-ethylhexyl)ester,
   4-Methoxyzimtsäureisopentylester;
- Ester der Salicylsäure, vorzugsweise
   Salicylsäure(2-ethylhexyl)ester,
   Salicylsäure(4-isopropylbenzyl)ester,
   Salicylsäurehomomenthylester;
- Derivate des Benzophenons, vorzugsweise
   2-Hydroxy-4-methoxybenzophenon,
   2-Hydroxy-4-methoxy-4'-methylbenzophenon,
   2,2'-Dihydroxy-4-methoxybenzophenon;
- Ester der Benzalmalonsäure, vorzugsweise
   4-Methoxybenzalmalonsäuredi(2-ethylhexyl)ester;
- 2,4,6-Trianilino-(p-carbo-2'-ethyl-1'-hexyloxy)-1,3,5-triazin.

Vorteilhafte wasserlösliche UVB-Filtersubstanzen sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren
   Salze;
- Sulfonsäure-Derivate des 3-Benzylidencamphers, wie z.B.
   4-(2-Oxo-3-bornylidenmethyl)benzolsulfonsäure,
   2-Methyl-5-(2-oxo-3-bornylidenmethyl)sulfonsäure und deren Salze.

Die Liste der genannten UVB-Filter, die in Kombination mit den erfindungsgemäßen anorganischen Pigmenten verwendet werden können, soll selbstverständlich nicht limitierend sein.

Es kann auch von Vorteil sein, die erfindungsgemäßen anorganischen Pigmente mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist es vorteilhaft, die erfindungsgemäßen anorganischen Pigmente mit UVA- und UVB-Filtern zu kombinieren.

Gegenstand der Erfindung ist auch ein Verfahren zur Herstellung der erfindungsgemäßen topischen, kosmetischen und/oder dermatologischen Zubereitungen, das dadurch gekennzeichnet ist, daß man in an sich bekannter Weise das anorganische Pigment in der vorzugsweise flüssigen Lipidphase, in welche gegebenenfalls ein Verdickungsmittel eingearbeitet worden ist, bei gleichmäßigem Rühren und gegebenenfalls unter Erwärmen suspendiert und gewünschtenfalls homogenisiert, hernach die Suspension mit der wäßrigen Phase, in welche gegebenenfalls ein Verdickungsmittel und/oder anorganisches Pigment eingearbeitet worden ist, und welche vorzugsweise etwa die gleiche Temperatur besitzt wie die Suspension, vermischt, gewünschtenfalls homogenisiert und auf Raumtemperatur abkühlen läßt. Nach Abkühlen auf Raumtemperatur kann, insbesondere, wenn noch flüchtige Bestandteile eingearbeitet werden sollen, nochmaliges Homogenisieren erfolgen.

Die vorliegende Erfindung umfaßt auch ein Verfahren zum Schutze der Haut und der Haare vor Nesseltieren und deren Nesselgift und vor UV-Strahlung, das dadurch gekennzeichnet ist, daß man eine topische, kosmetische und/oder dermatologische Formulierung, welche eine wirksame Konzentration an anorganischen Mikropigmenten sowie gegebenenfalls zusätzlichen organischen UV-Filtersubstanzen enthält, wobei die Formulierungen Hydrodispersionen, welche aus einer inneren Lipidphase und einer äußeren wäßrigen Phase bestehen, und welche im wesentlichen frei von Emulgatoren sind, darstellen und die anorganischen Mikropigmente in die Lipidphase der Hydrodispersionen eingearbeitet sind, in ausreichender Menge auf die Haut oder Haare aufbringt, sowie die Verwendung dieser Formulierungen insbesondere für diese Zwecke.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken.

### Beispiel 1

| Sonnengel, Lichtschutzfaktor 12 | |
|---|---|
| | Gew.-% |
| Carbomer 980 und 981 (Gew.-Verhältnis 10:1) | 1,0 |
| Octylmethoxycinnamat | 6,0 |
| TiO₂, Partikelgröße < 100 nm, hydrophobiert | 3,0 |
| Ethanol | 5,0 |
| Butylenglycol | 3,0 |
| EDTA-Lösung (14%-ig), | 0,5 |
| Phenyltrimethicon | 3,0 |
| Butylmethoxydibenzoylmethan | 2,0 |
| Methylbenzylidencampher | 1,5 |
| Tocopherylacetat | 1,0 |
| Hyaluronsäure | 0,03 |
| Tromethamin (in einer Menge zur pH-Einstellung auf | 5,0 - 5,5) |
| Xanthangummi | 0,3 |
| Parfüm, Konservierungsmittel, Farbstoffe | q.s. |
| Wasser | ad 100,00 |

### Beispiel 2

| Sonnengel, Lichtschutzfaktor 12 | |
|---|---|
| | Gew.-% |
| Phenyltrimethicon | 4,50 |
| Carbomer 981 | 1,50 |
| Octylmethoxycinnamat | 7,50 |
| Parsol ^{R} 1789 | 3,00 |
| TiO₂, Partikelgröße < 100 nm | 4,50 |
| Ethanol | 9,00 |
| Glycerin | 4,50 |
| Hydroxypropylmethylcellulose | 0,30 |
| EDTA-Lösung (14 %-ig) | 0,75 |
| Trisaminopromethamin | 2,01 |
| Parfum, Konservierungsmittel, Farbstoffe | q.s. |
| Wasser, VES | ad 100,00 |

### Beispiel 3

| Sonnengel, Lichtschutzfaktor 12 | |
|---|---|
| | Gew.-% |
| Octyldodecanol | 4,50 |
| Carbomer 981 | 1,50 |
| Octylmethoxycinnamat | 7,50 |
| Parsol ^{R} 1789 | 3,00 |
| TiO₂, Partikelgröße < 100 nm | 4,50 |
| Ethanol | 9,00 |
| Butylenglycol | 4,50 |
| Hydroxypropylmethylcellulose | 0,30 |
| EDTA-Lösung (14 %-ig) | 0,75 |
| Trisaminopromethamin | 2,01 |
| Parfum, Konservierungsmittel, Farbstoffe | q.s. |
| Wasser, VES | ad 100,00 |

### Beispiel 4

| Sonnengel, Lichtschutzfaktor 12 | |
|---|---|
| | Gew.-% |
| Ricinusöl | 4,50 |
| Carbomer 981 | 1,50 |
| Octylmethoxycinnamat | 7,50 |
| Parsol ^{R} 1789 | 3,00 |
| TiO₂, Partikelgröße < 100 nm | 4,50 |
| Ethanol | 9,00 |
| Butylenglycol | 4,50 |
| Hydroxypropylmethylcellulose | 0,30 |
| EDTA-Lösung (14 %-ig) | 0,75 |
| Trisaminopromethamin | 2,01 |
| Parfum, Konservierungsmittel, Farbstoffe | q.s. |
| Wasser, VES | ad 100,00 |

### Versuchsbericht

Die Schutzwirkung der erfindungsgemäßen Zubereitungen gegenüber Quallen wird mit lebenden Feuerquallen (Cyanea capillata) an Schweinehautproben nachgewiesen. Dazu werden Präparatmengen der Formulierung des Beispiels 1 entsprechend den Empfehlungen der Europäischen Lichtschutzkommission homogen auf den Hautproben verteilt. Nach 10 Minuten werden die Proben 4 s lang mit den Tentakeln in Kontakt gebracht. Die Proben werden dann rasterelektronenmikroskopisch untersucht und die Nesselkapselanzahl sowie die Präparathaftung werden bestimmt. Es zeigt sich, daß die erfindungsgemäße Zubereitung gut auf der Haut haftet und praktisch keine Nesselkapseln ausgelöst werden.

Die erfindungsgemäßen Zubereitungen besitzen eine hervorragende Schutzwirkung.

## Patentansprüche

1. Verwendung von topischen Formulierungen mit einem Gehalt an
a) anorganischen Mikropigmenten sowie
b) gegebenenfalls zusätzlichen organischen UV-Filtersubstanzen,
c) wobei die Formulierungen Hydrodispersionen darstellen,
d) welche aus einer inneren Lipidphase und einer äußeren wäßrigen Phase bestehen, und
e) welche im wesentlichen frei von Emulgatoren sind, und
f) wobei die anorganischen Mikropigmente bevorzugt in die Lipidphase der Hydrodispersionen eingearbeitet sind,
zum Schutz gegen Nesseltiere.

2. Topische Formulierungen mit einem Gehalt an
a) anorganischen Mikropigmenten sowie
b) gegebenenfalls zusätzlichen organischen UV-Filtersubstanzen,
c) wobei die Formulierungen Hydrodispersionen darstellen,
d) welche aus einer inneren Lipidphase und einer äußeren wäßrigen Phase bestehen, und
e) welche im wesentlichen frei von Emulgatoren sind, und
f) wobei die anorganischen Mikropigmente bevorzugt in die Lipidphase der Hydrodispersionen eingearbeitet sind, zum Schutz gegen Nesseltiere.

3. Verwendung von Formulierungen nach Anspruch 1, dadurch gekennzeichnet, daß die anorganischen Pigmente auf den Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle bzw. Abmischungen aus solchen Oxiden basieren.

4. Verwendung von Formulierungen nach Anspruch 1, dadurch gekennzeichnet, daß die anorganischen Pigmente oberflächlich hydrophobisiert sind.

5. Verwendung von Formulierungen nach Anspruch 1, dadurch gekennzeichnet, daß die wäßrige Phase der Hydrodispersionen ein Verdickungsmittel enthält, insbesondere ein Polyacrylat.

6. Verwendung von Formulierungen nach Anspruch 1, dadurch gekennzeichnet, daß sie 0,01 Gew.-% bis 30 Gew.-%, insbesondere 0,1 Gew.-% bis 6 Gew.-%, bezogen auf das Gesamtgewicht der Formulierungen, an anorganischen Pigmenten enthalten.

7. Verfahren zur Herstellung von topischen, kosmetischen und/oder dermatologischen Zubereitungen gemäß Anspruch 1, dadurch gekennzeichnet, daß man in an sich bekannter Weise ein anorganisches Pigment in einer vorzugsweise flüssigen Lipidphase, in welche gegebenenfalls ein Verdickungsmittel und/oder anorganisches Pigment eingearbeitet worden ist, bei gleichmäßigem Rühren und gegebenenfalls unter Erwärmen suspendiert und gewünschtenfalls homogenisiert, hernach die Suspension mit einer wäßrigen Phase, in welche gegebenenfalls ein Verdickungsmittel eingearbeitet worden ist, und welche vorzugsweise etwa die gleiche Temperatur besitzt wie die Suspension, vermischt, gewünschtenfalls homogenisiert und auf Raumtemperatur abkühlen läßt und gegebenenfalls nach Abkühlen auf Raumtemperatur, insbesondere, wenn noch flüchtige Bestandteile eingearbeitet werden sollen, nochmaliges Homogenisieren erfolgen läßt.

8. Verfahren zum Schutze der Haut und der Haare vor Nesseltieren und deren Nesselgift und vor UV-Strahlung, dadurch gekennzeichnet, daß man eine topische, kosmetische und/oder dermatologische Formulierung, welche eine wirksame Konzentration an anorganischen Mikropigmenten als UV-Filtersubstanzen sowie gegebenenfalls zusätzlichen organischen UV-Filtersubstanzen enthält, wobei die Formulierungen Hydrodispersionen, welche aus einer inneren Lipidphase und einer äußeren wäßrigen Phase bestehen, und welche im wesentlichen frei von Emulgatoren sind, darstellen und die anorganischen Mikropigmente in die Lipidphase der Hydrodispersionen eingearbeitet sind, in ausreichender Menge auf die Haut oder Haare aufbringt.
